# FASCICULE DE BREVET EUROPEEN

(11) **EP 2 687 189 B1**
(45) Date de publication et mention de la délivrance du brevet: **14.10.2015**
(21) Numéro de dépôt: 13176498.7
(22) Date de dépôt: 15.07.2013
(51) Int. Cl.: A61F 2/30, A61F 2/42

(54) **Implant d'interposition trapézien**
Trapezbeinimplantat zum Einfügen
Interpositional trapezium implant

(30) Priorité: 16.07.2012 FR 1256863
(43) Date de publication de la demande: 22.01.2014
(73) Titulaire: Tornier, 38330 Montbonnot-Saint-Martin (FR)
(72) Inventeur: Hassler, Michel, 38330 SAINT ISMIER (FR); Bellemere, Philippe, 44000 NANTES (FR)
(74) Mandataire: Grand, Guillaume

(56) Documents cités:
- EP-A1- 2 263 614
- WO-A1-98/47449
- WO-A1-2006/000890
- FR-A3- 2 957 780
- US-A1- 2006 241 777
- US-A1- 2009 254 190
- US-B1- 6 436 146

## Description

La présente invention concerne un implant d'interposition trapézien.

Comme son nom l'indique, un implant trapézien est destiné à être implanté en lieu et place du trapèze de la main après que cet os ait été retiré au cours d'une intervention dite de trapézectomie. Une telle trapézectomie est pratiquée lorsqu'au moins une des articulations du trapèze avec le premier métacarpien, le scaphoïde et le trapézoïde est endommagée par un processus pathologique dégénératif ou inflammatoire, typiquement de l'arthrose. Ceci étant dit, la mise en oeuvre de l'invention n'est pas limitée à un tableau clinique spécifique mais s'intéresse, plus globalement, aux traitements chirurgicaux de la main, incluant une trapézectomie totale.

En revanche, on notera que l'invention ne concerne pas les traitements chirurgicaux n'impliquant qu'une trapézectomie partielle, c'est-à-dire une trapézectomie réséquant uniquement une partie du trapèze, notamment celle dirigée vers le métacarpien, tandis que le reste du trapèze est préservé, notamment au niveau de son articulation avec le scaphoïde et de son articulation avec le trapézoïde. A titre d'information relevant donc d'un domaine distinct de celui de la présente invention, le lecteur pourra se reporter à EP-A-2 263 614 qui divulgue un implant comportant un corps définissant une surface articulaire métacarpienne et une surface articulaire opposée, qui sont situées de part et d'autre d'un plan médian du corps et qui correspondent chacune à un segment axial de cylindre, de cône ou de tore, ces deux segments axiaux de cylindre, de cône et/ou de tore étant centrés sur des axes respectifs qui, projetés sur le plan médian, sont sensiblement perpendiculaires l'un à l'autre.

La trapézectomie totale est aujourd'hui largement pratiquée sur des indications d'arthrose péri-trapézienne, c'est-à-dire d'arthrose intéressant les différentes surfaces articulaires du trapèze, mais aussi sur des indications d'arthrose isolée, telle qu'une arthrose de l'articulation trapézo-métacarpienne, autrement appelée rhizarthrose. La trapézectomie permet au premier métacarpien de tourner dans le vide créé par l'absence de l'os du trapèze. Cependant, si aucun geste n'y est associé, la colonne du pouce de la main se rétracte sous l'effet de la traction des ligaments et un collapsus finit par empêcher cette rotation et engendrer des douleurs par arthrose secondaire. Pour remédier à cet inconvénient, la trapézectomie totale est fréquemment associée à une ligamentoplastie, le cas échéant associée à la pose, en lieu et place du trapèze, d'un anchois soit ligamentaire, en étant prélevé sur le patient lui-même, soit réalisé en fibres synthétiques, afin d'occuper l'espace libéré par le trapèze retiré et éviter ainsi la descente de la base du pouce. Cependant, les résultats cliniques de tels ligamentoplasties restent mitigés : délai de récupération fonctionnelle très long, raccourcissement disgracieux de la colonne du pouce avec hyper-extension compensatrice de l'articulation métacarpo-phalangienne, diminution de la force de serrage par le pouce, et dégradation à plus ou moins long terme du résultat par l'apparition d'un conflit métacarpo-trapézoïdien et/ou métacarpo-scaphoïdien du fait du raccourcissement de la loge trapézienne.

Les inconvénients listés ci-dessus visent à être corrigés en associant la trapézectomie à une pose d'un implant d'interposition trapézien. Historiquement, les premiers implants trapéziens sont des implants monoblocs en silicone, incluant une partie cylindrique remplaçant le trapèze, dont une extrémité concave s'appuie sur le scaphoïde et dont l'extrémité opposée est pourvue d'une queue conique de stabilisation, à insérer dans le métacarpe. Ce type d'implant d'interposition est aujourd'hui abandonné en raison des échecs liés aux débris de silicone provoquant des « siliconites » avec une résorption osseuse importante, des fractures de l'implant et/ou des problèmes d'instabilité de l'implant. Il a bien été proposé de réaliser cet implant en titane, mais il induit alors une gêne et des douleurs pour le patient.

Plus récemment, il a été proposé de conformer des implants trapéziens de manière identique à un os de trapèze naturel. Ces implants imitant la nature ont d'ailleurs été déclinés en plusieurs matériaux constitutifs. Ces implants ont tous connu des échecs cliniques importants, liés au fait que lorsqu'une arthrose est à un tel point d'avancement qu'elle conduit à pratiquer une trapézectomie, les tissus environnants le trapèze retiré sont tellement déformés qu'il est impossible d'y mettre en place de manière stable un implant trapézien imitant la nature. Même en leur adjoignant des moyens de stabilisation, ces implants sont particulièrement douloureux pour les patients en raison des surcontraintes qu'ils induisent dans la main. Des exemples de tels implants sont fournis par US-A-2006/0241777 et US-A-2009/0254190.

On connaît par ailleurs par US-B-6 436 146 un implant d'interposition trapézien, constitué d'un corps monobloc sphérique ou ellipsoïdal, mis en place dans la loge trapézienne. Cet implant a résolu le problème de la rétractation de la colonne du pouce, ainsi que les problèmes de durée de récupération liés à la trapézectomie. Cependant, eu égard à sa forme convexe, cet implant doit être mis en place selon une technique chirurgicale délicate, basée sur une médialisation de l'implant : en effet, l'implant doit être poussé dans un « cul de sac » préparé par le chirurgien par résection partielle du trapézoïde, assurant alors la stabilité de l'implant.

Le but de la présente invention est de proposer un implant trapézien dont l'implantation est facile et pérenne, y compris dans le cas du traitement d'arthroses du trapèze à divers stades d'évolution.

A cet effet, l'invention a pour objet un implant d'interposition trapézien, tel que défini à la revendication 1.

Conformément à une méthode chirurgicale de pose d'un implant d'interposition trapézien tel que défini ci-dessus :
- on pratique une trapézectomie totale, et
- on interpose le corps de l'implant entre le métacarpien et le scaphoïde de manière que l'axe associé à la surface articulaire scaphoïdienne s'étend dans l'un des plans antéropostérieur et frontal du patient, tandis que l'axe associé à la surface articulaire métacarpienne s'étend dans l'autre de ces plans.

Une des idées à la base de l'invention est d'interposer entre le métacarpien et le scaphoïde un corps volumineux non pas sphérique ou ellipsoïdal, mais un corps présentant des surfaces articulaires opposées, qui sont concaves au moins dans une direction et contre lesquelles vont s'appuyer pour rouler et glisser les surfaces osseuses corticales, revêtues ou non de cartilage selon leur état pathologique, du métacarpien et du scaphoïde. Pour obtenir un comportement articulaire proche du comportement anatomique du trapèze, ainsi qu'une bonne mobilité du scaphoïde contre l'implant dans des mouvements d'inclinaison radiale et cubitale, ces deux surfaces articulaires sont réalisées par des segments axiaux de cylindre, de cône et/ou de tore, dont les axes centraux ou, plus généralement, pour tenir compte du fait que l'axe central d'un segment axial de tore est courbe, les projections de ces axes centraux sur le plan médian de l'implant sont sensiblement perpendiculaires l'une à l'autre : le corps de l'implant conforme à l'invention dissocie ainsi une interface de type selle de cheval en les deux surfaces articulaires précitées. D'un point de vue cinématique, ces deux surfaces articulaires orthogonales autorisent une mobilité pivotante autour des deux axes centraux précités. De plus, la courbure de ces deux surfaces articulaires stabilise et aligne le corps de l'implant entre le métacarpien et le scaphoïde, ce qui évite de prévoir des moyens de fixation osseuse. En outre, l'invention présente l'avantage de maximiser l'étendue du contact entre le corps de l'implant et les os du métacarpien et du scaphoïde, au niveau de ces deux surfaces articulaires. Il en résulte que l'implant ne s'enfonce pas dans les os, préservant ainsi sa mobilité, sans pour autant qu'il soit nécessaire que ces surfaces reproduisent rigoureusement l'anatomie naturelle d'origine de l'os du trapèze.

En pratique, le corps de l'implant selon l'invention présente une épaisseur non négligeable puisqu'il comble la loge trapézienne, libérée de son trapèze après que la trapézectomie totale a été pratiquée. Bien entendu, une gamme de quelques épaisseurs peut avantageusement être proposée pour choisir un implant adapté à la taille morphologique du patient à traiter. L'épaisseur du corps de l'implant vise à éviter la descente du pouce, autrement à maintenir la hauteur de la colonne du pouce à un niveau compatible avec les structures des tissus environnants. De plus, cette épaisseur, combinée à la conformation des deux surfaces articulaires du corps de l'implant, permet de limiter fortement, voire le plus souvent d'éviter les préparations osseuses des surfaces corticales du métacarpien et du scaphoïde. Dans tous les cas, la taille et la forme de l'implant selon l'invention permettent, d'une part, ne pas avoir à creuser ou à passer à travers les surfaces corticales du métacarpien et du scaphoïde et, d'autre part, de stabiliser immédiatement son corps essentiellement, voire exclusivement par la contrainte de l'enveloppe capsulaire et ligamentaire autour de la loge trapézienne, sans pour autant que ce corps subisse une hypercontrainte puisqu'il doit conserver une certaine liberté afin que sa position s'adapte en fonction des contraintes de cette enveloppe, liées aux différents mouvements possibles de la main. La stabilité du corps de l'implant est renforcée en ceinturant une portion de la périphérie du corps par une languette tendineuse du patient, reçue de manière glissante dans une gorge périphérique ad hoc du corps, creusée dans l'épaisseur de celui-ci.

Des caractéristiques additionnelles avantageuses de l'implant conforme à l'invention, sont spécifiées aux revendications dépendantes.

L'invention sera mieux comprise à la lecture de la description qui va suivre, donnée uniquement à titre d'exemple et faite en se référant aux dessins sur lesquels :
- la figure 1 est une vue en élévation représentant certains des constituants osseux d'une main humaine droite, dans laquelle est à implanter un implant conforme à l'invention ;
- la figure 2 est une vue en perspective d'un implant conforme à l'invention ;
- la figure 3 est une vue en élévation selon la flèche III de la figure 2 ;
- les figures 4 et 5 sont des coupes selon respectivement les lignes IV-IV et V-V de la figure 3, montrant également en partie les os de la main après mise en place de l'implant ;
- la figure 6 est une coupe selon la ligne VI-VI de la figure 4 ;
- la figure 7 est un schéma illustrant la géométrie de construction des surfaces articulaires de l'implant des figures 1 à 6 ; et
- les figures 8 à 10 sont des vues analogues à la figure 7, illustrant respectivement des variantes pour la géométrie de construction des surfaces articulaires de l'implant conforme à l'invention.

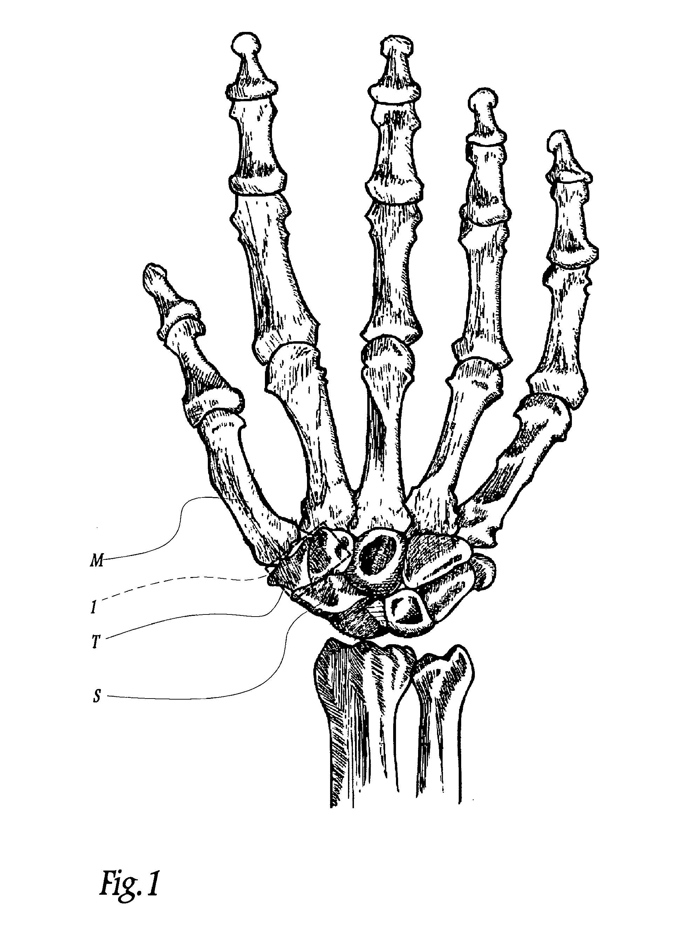

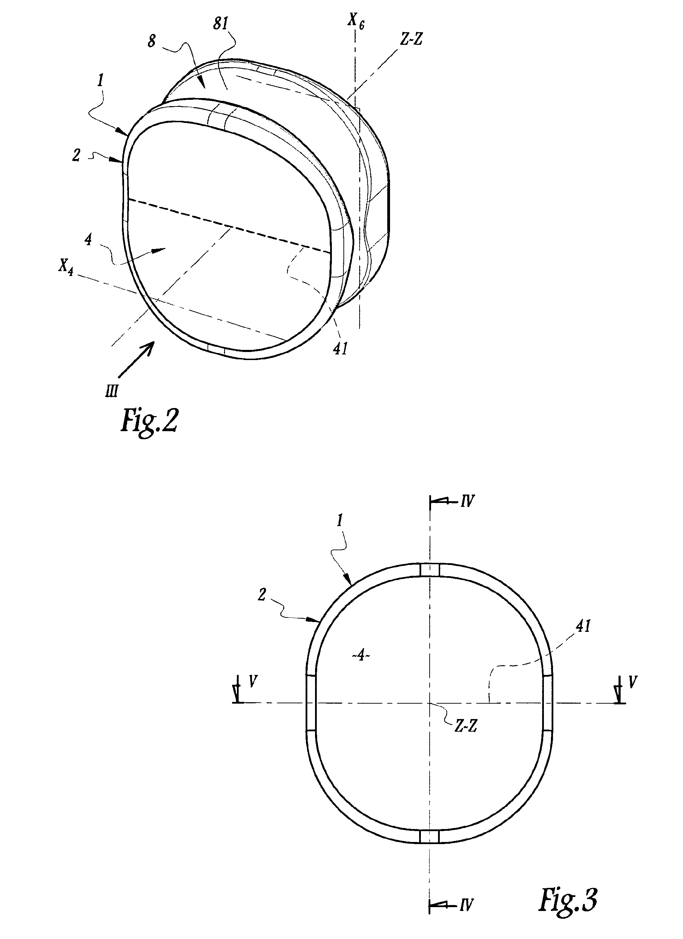

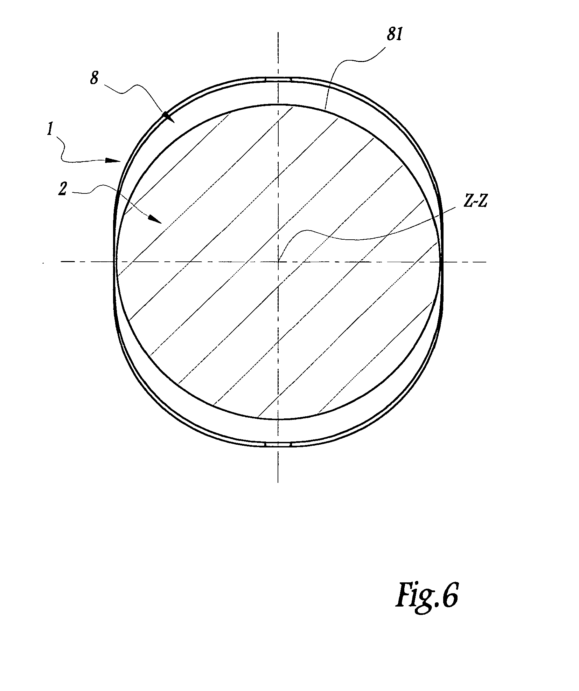

Sur les figures 2 à 6 est représenté un implant trapézien 1, qui, comme montré schématiquement en pointillés sur la figure 1, est adapté pour être implanté de manière interposée entre le scaphoïde S et le premier métacarpien M d'une main humaine, telle que celle montrée sur la figure 1 à titre d'exemple.

L'implant 1 comporte un corps 2 globalement discoïdal, centré sur un axe géométrique référencé Z-Z. Le corps 2 délimite ainsi, d'une part, deux surfaces principales 4 et 6 opposées selon l'axe Z-Z, qui sont séparées l'une de l'autre par l'épaisseur, selon cet axe Z-Z, du corps 2, et, d'autre part, une surface périphérique 8, qui relie l'une à l'autre les surfaces 4 et 6 et qui court sur toute la périphérie du corps 2 autour de l'axe Z-Z, en présentant, dans l'exemple de réalisation considéré ici, une étendue selon cet axe Z-Z qui est sensiblement constante. Observé selon l'axe Z-Z, le corps 2 présente un contour global extérieur rectangulaire aux coins émoussés. En variantes non représentées, d'autres formes sont envisageables pour ce contour, telles qu'un carré, un rond, une ellipse, etc.

Dans la forme de réalisation envisagée sur les figures, l'implant 1 n'est constitué que du corps 2, dans le sens où l'implant ne comporte aucun autre composant, notamment aucun composant de fixation osseuse qui permettrait d'ancrer fixement le corps 2 dans le métacarpien M ou dans le scaphoïde S. Le corps 2 constitue ainsi un implant exclusivement d'interposition de manière que ce corps présente, après implantation entre le métacarpien et le scaphoïde, une certaine liberté de mouvement, sa position s'adaptant aux contraintes qui lui sont appliquées, en fonction des mouvements de la main, en particulier des mouvements d'articulation entre l'implant et les os du métacarpien et du scaphoïde.

Dans cette optique, le corps 2 est avantageusement réalisé d'un seul tenant en un bloc de graphite recouvert de carbone pyrolytique, ce qui confère à ce corps une bonne biocompatibilité, une haute résistance mécanique et une bonne tenue à l'usure vis-à-vis des os du métacarpien et du scaphoïde. En pratique, d'autres matériaux peuvent être utilisés, notamment un alliage métallique à base de chrome et de cobalt, une matière plastique, comme le polyéthylène et le PEEK, du silicone, de la céramique, une matière polymère visco-élastique, etc.

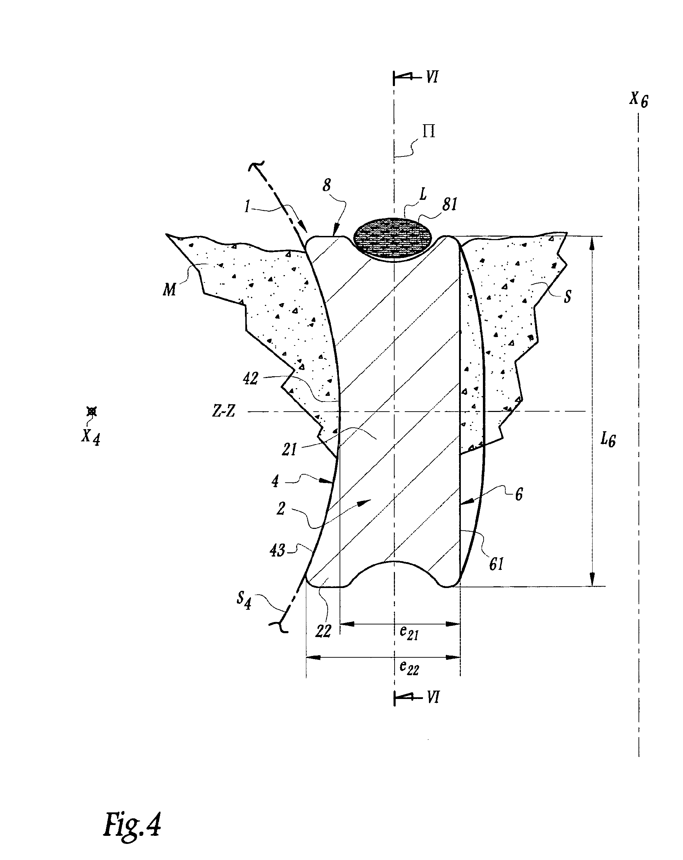

La surface 4 est concave. Elle correspond géométriquement à une portion de cylindre, qui est centrée sur un axe X₄ et dont la section transversale S₄, qui est constante suivant l'axe X₄ et qui correspond donc à la directrice de la surface 4, est sensiblement elliptique, comme bien visible sur la figure 4. L'axe X₄ s'étend de manière perpendiculaire à l'axe Z-Z, ces axes étant agencés de manière séquente dans l'exemple de réalisation considéré ici.

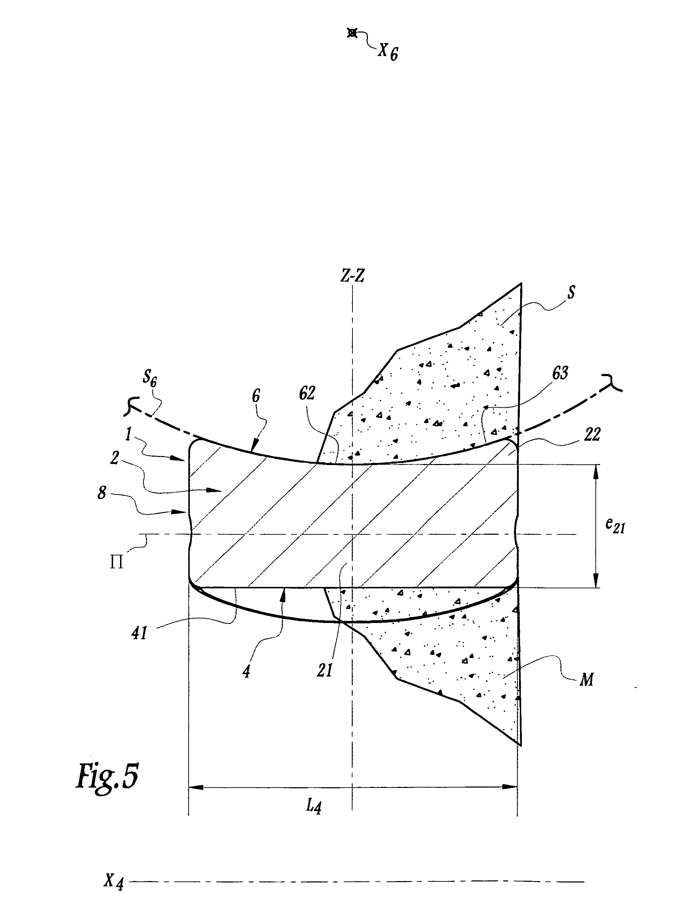

De même, la surface 6 est concave et correspond à une portion de cylindre, dont la section transversale S₆ est sensiblement elliptique et qui est centrée sur un axe X₆ perpendiculaire, ici de manière séquente, avec l'axe Z-Z, comme bien visible sur la figure 5.

Ainsi, les axes X₄ et X₆ s'étendent de manière perpendiculaire l'un à l'autre, de manière non séquente. En particulier, les lignes de fond 41 et 61, qui appartiennent respectivement aux surfaces 4 et 6 et qui s'étendent respectivement parallèlement aux axes X₄ et X₆, s'étendent de manière perpendiculaire l'une à l'autre, en étant séparées, selon l'axe Z-Z, par la partie centrale 21 du corps 2. Autrement dit, dans un plan médian π du corps 2, qui est perpendiculaire à l'axe Z-Z et de part et d'autre duquel sont situées les surfaces 4 et 6, les projections respectives de ces axes X₄ et X₆ sont perpendiculaires et sécantes.

De par les caractéristiques géométriques des surfaces 4 et 6, on comprend que, en coupe transversale incluant l'axe Z-Z, le corps 2 présente une épaisseur variable, en étant minimale dans la partie centrale 21 du corps tandis qu'elle est progressivement croissante en s'éloignant de cette partie centrale 21, jusque dans la partie périphérique 22 du corps 2, comme bien visible sur les figures 4 et 5.

Par ailleurs, à titre d'aménagement dont l'intérêt apparaîtra plus loin, la surface périphérique 8 du corps 2 n'est pas pleine, mais est creusée d'une gorge 81 qui court en longueur sur la périphérie du corps 2, autour de l'axe Z-Z. Plus précisément, dans l'exemple de réalisation considéré ici, la profondeur de cette gorge 81 n'est pas constante sur toute la périphérie du corps 2, mais varie de manière continue entre une valeur maximale, qui est atteinte dans les deux portions de la surface 8, diamétralement opposées l'une à l'autre suivant la direction de l'axe X₆, et une valeur minimale, qui est atteinte dans les deux portions de la surface 8, qui sont opposées l'une à l'autre suivant la direction de l'axe X₄, comme bien visible par comparaison des figures 4 et 5.

D'autres caractéristiques de l'implant 1 apparaitront ci-après, dans le cadre de la description d'un exemple de mise en place de cet implant entre le métacarpien M et le scaphoïde S montrés à la figure 1.

Dans un premier temps opératoire, un chirurgien accède au trapèze T entre le métacarpien M et le trapèze T, via une voie d'abord postérieure ou antérieure. Pour accéder à la loge trapézienne entre le métacarpien et le scaphoïde, la capsule articulaire et les ligaments entourant cette loge sont partiellement incisés, étant entendu que cette capsule et ses ligaments seront reconstruits en fin d'intervention.

Dans un deuxième temps opératoire, le chirurgien réalise une trapézectomie totale, en pratiquant des gestes chirurgicaux connus, qui ne seront donc pas détaillés davantage. Le chirurgien libère ainsi les surfaces osseuses délimitées par le métacarpien M et le scaphoïde S, qui, jusqu'alors, étaient appliquées contre le trapèze T. Le chirurgien peut alors préparer le matacarpien si besoin, notamment en coupant les crêtes et les berges dorsales et palmaires de la tête du métacarpien. Quant au scaphoïde, aucune préparation de ce type n'est a priori nécessaire. Par ailleurs, le cas échéant, le chirurgien peut débarasser l'une et/ou l'autre des surfaces osseuses précitées d'ostéophytes.

Dans un troisième temps opératoire, si besoin en exerçant une traction relative entre le métacarpien M et le scaphoïde S selon la direction longitudinale du métacarpien, le chirurgien positionne l'implant 1 dans la loge trapézienne séparant le métacarpien et le scaphoïde, en interposant le corps 2 entre ces deux os de manière que la surface 4 est tournée vers le métacarpien, avec l'axe X₄ s'étendant dans un plan frontal, tandis que la surface 6 est tournée vers le scaphoïde, avec l'axe X₆ qui s'étend dans un plan antéro-postérieur, étant entendu que les termes « frontal », « antéro-postérieur » et similaires s'entendent ici dans leur sens anatomique, par rapport au patient dont la main est opérée. En variante non représentée, l'implant 1 est mis en place, entre le métacarpien M et le scaphoïde S, dans une configuration basculée de 90° autour de l'axe Z-Z : une préparation préalable des extrémités du métacarpien et du scaphoïde peut être nécessaire, mais cette variante illustre la capacité du corps 2 à être implanté de façon que ses surfaces articulaires 4 et 6 s'adaptent au mieux aux extrémités du métacarpien et du scaphoïde du patient opéré, selon l'état initial de ces extrémités osseuses et/ou pour minimiser la profondeur d'entame éventuelle de ces extrémités lors de la chirurgie.

Une fois que l'implant 1 est ainsi positionné entre le métacarpien M et le scaphoïde S, et que la traction axiale précitée est relâchée, le corps 2 de l'implant 1 se trouve retenu de manière mobile entre le métacarpien et le scaphoïde, sous la contrainte de la capsule et des ligaments entourant la loge trapézienne, autrement dit sous une contrainte dont la résultante est sensiblement alignée avec l'axe Z-Z.

Le chirurgien peut ensuite refermer les tissus mous autour de l'implant, le cas échéant par reconstruction, voire moyennant une ligamentoplastie. En particulier, une telle ligamentoplastie est mise en oeuvre en utilisant la gorge périphérique 81 : pour ce faire, le chirurgien ceinture partiellement le corps 2 avec un tendon lié ou appartenant aux tissus mous environnants. Suivant une mise en oeuvre préférée, le tendon précité est soit constitué d'une languette tendineuse venant du muscle fléchisseur radial du carpe, couramment appelé muscle FCR (acronyme de « flexor carpi radialis »), cette languette s'étendant alors, autour d'une portion périphérique du corps 2 de l'implant, depuis le dos du pouce jusqu'à être fixé à la base palmaire de la capsule entourant la loge trapézienne, soit constitué d'une languette tendineuse venant du muscle long abducteur du pouce, couramment appelé muscle APL (acronyme de « abductor pollisis longus »), cette languette entourant partiellement le corps de l'implant, en s'étendant depuis la base palmaire du pouce, jusqu'à être attachée au côté dorsal de la capsule. A titre d'exemple, sur la figure 4, une des languettes tendineuses précitées est représentée sous la référence L, en étant reçue dans l'une des portions de la gorge 81, présentant la profondeur la plus importante. Dans tous les cas, on comprend que la présence d'un ligament ou similaire, courant sur une portion seulement de la périphérie du corps 2, en étant reçu de manière glissante dans le fond de la portion correspondante de la gorge 81, permet de renforcer la stabilité de l'implant dans la loge trapézienne, ce tendon ou similaire cravatant ainsi l'implant sur une portion périphérique de sa surface 8, préférentiellement sur son bord antéro-externe, étant remarqué que, lors des mouvements du pouce, ce tendon ou similaire glisse, suivant sa direction longitudinale, contre le fond de la gorge 81.

La partie du pouce ainsi munie de l'implant 1 retrouve un comportement cinématique proche, voire quasi-identique aux comportements anatomiques d'origine de cette partie du pouce. En effet, le métacarpien M s'articule alors contre la surface 4, essentiellement par basculement autour de l'axe X₄, tandis que, dans le même temps, le scaphoïde S s'articule contre la surface 6, essentiellement par basculement autour de l'axe X₆. Grâce à l'implant 1, le métacarpien M et le scaphoïde S sont articulés l'un vis-à-vis de l'autre, autour des deux axes perpendiculaires X₄ et X₆ en quelque sorte à la façon d'un joint de cardan à deux axes d'articulation distants. Cette cinématique reproduit efficacement les mobilités des articulations naturelles sensiblement sellaires entre le métacarpien et le trapèze et entre le trapèze et le scaphoïde. De plus, on notera que les surfaces 4 et 6 s'appuient alors contre les couches corticales osseuses respectives du métacarpien M et du scaphoïde S, ce qui évite que le corps 2 ne s'enfonce dans l'un et/ou l'autre de ces os. La mobilité fournie par l'implant 1 est ainsi pérenne.

Avantageusement, pour favoriser les mouvements de roulement et de glissement du métacarpien M et du scaphoïde S contre respectivement les parties centrales 42 et 62 des surfaces 4 et 6, la section elliptique S₄, S₆ de ces surfaces présente, dans ces régions centrales 42 et 62, une courbure plus petite que le reste de la section. Autrement dit, les régions centrales 42 et 62 des surfaces 4 et 6 sont alors un peu plus aplaties, c'est-à-dire un peu moins courbées que le reste de ces surfaces.

Par ailleurs, en service, le corps 2 est stabilisé entre le métacarpien M et le scaphoïde S du fait de la courbure elliptique des sections respectives S₄, S₆ des surfaces 4 et 6. Avantageusement, pour renforcer cette stabilité, la section des régions périphériques respectives 43 et 63 des surfaces 4 et 6 présente une courbure plus grande que le reste de cette section : en exagérant ainsi la courbure des surfaces 4 et 6 au niveau de leur bordure d'extrémité périphérique, la coopération surfacique du corps 2 et des os du métacarpien M et du scaphoïde S auto-stabilise l'implant 1. De surcroît, les régions périphériques 43 et 63 des surfaces 4 et 6 peuvent ainsi compenser l'usure périphérique, liée à l'arthrose, des extrémités osseuses du métacarpien et du scaphoïde.

En pratique, on comprend que, dans la mesure où le corps 2 est un implant trapézien, son épaisseur suivant l'axe Z-Z est significative, dans le sens où la présence de ce corps doit maintenir l'écartement entre le métacarpien M et le scaphoïde S : aussi, à titre de quantification, l'épaisseur minimale e₂₁ du corps 2, c'est-à-dire son épaisseur dans sa région centrale 21, autrement dit entre les régions centrales 42 et 62 des surfaces 4 et 6, est prévue supérieure ou égale à 5 mm. Ceci étant, dans la mesure où le corps 2 est un implant d'interposition, on comprend également que son épaisseur est limitée, dans le sens où la présence de ce corps ne doit pas entraîner une hypercontrainte de la loge trapézienne. Ainsi, à titre d'exemple numérique, l'épaisseur maximale e₂₂ du corps 2, c'est-à-dire son épaisseur dans sa partie périphérique 22, entre les régions périphériques 43 et 63 des surfaces 4 et 6, est prévue inférieure à 15 mm.

De même, pour s'adapter au mieux à la loge trapézienne entre le métacarpien M et le scaphoide S, la surface 6 présente avantageusement une dimension L₆, suivant son axe X₆, supérieure à la dimension L₄, suivant l'axe X₄, de la surface 4.

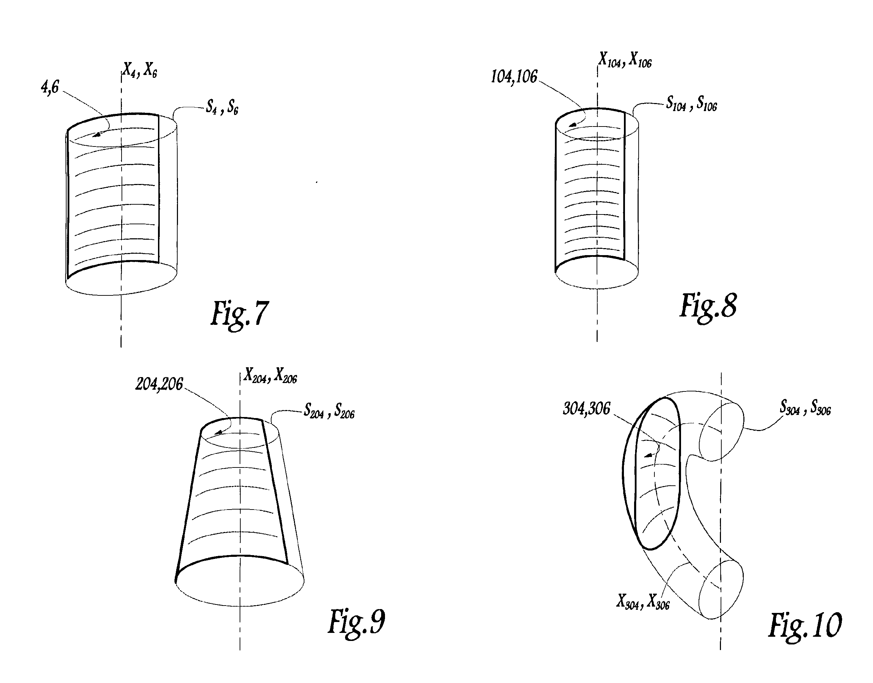

Par ailleurs, la géométrie des surfaces articulaires métacarpienne et scaphoïdienne peut être construite de manière différente de celle des surfaces 4 et 6 décrites jusqu'ici. Pour bien comprendre cet aspect de l'invention, on a représenté, sur la figure 7, un schéma permettant de visualiser la géométrie de construction des surfaces 4 et 6 : comme bien visible sur cette figure 7, chaque surface 4, 6 correspond à une portion, autrement dit à un segment axial, d'un cylindre d'axe central X₄, X₆, à base elliptique, c'est-à-dire avec sa section S₄, S₆ en forme d'ellipse centrée sur l'axe X₄, X₆. Ainsi, on retrouve bien ici la définition géométrique donnée plus haut pour les surfaces 4 et 6.

Sur les figures 8, 9 et 10 sont représentés des schémas similaires à celui de la figure 7, de manière à illustrer des variantes pour les surfaces articulaires 4 et 6 de l'implant 1. Ainsi, sur la figure 8, on propose des surfaces articulaires métacarpienne 104 et scaphoïdienne 106, qui correspondent chacune à un segment axial d'un cylindre, centré sur un axe X₁₀₄, X₁₀₆ et dont la section transversale S₁₀₄, S₁₀₆ est circulaire centrée sur l'axe précité. Autrement dit, les surfaces 4 et 6 et les surfaces 104 et 106 se distinguent uniquement par la forme géométrique de leur section transversale, à savoir elliptique pour les surfaces 4 et 6 et circulaire pour les surfaces 104 et 106.

De même, sur la figure 9, on propose des surfaces articulaires métacarpienne 204 et scaphoïdienne 206, qui correspondent chacune à un segment axial d'un cône, centré sur un axe X₂₀₄, X₂₀₆ et dont la section transversale S₂₀₄, S₂₀₆ est circulaire. A titre de variante non représentée, les sections précitées S₂₀₄ et S₂₀₆ peuvent être elliptiques.

Egalement de même, sur la figure 10, on propose des surfaces articulaires métacarpienne 304 et scaphoïdienne 306, qui correspondent chacune à un segment axial d'un tore, qui est associé à un axe central circulaire X₃₀₄, X₃₀₆ et dont la section transversale S₃₀₄, S₃₀₆, c'est-à-dire la section dans un plan perpendiculaire à l'axe précité, est circulaire centrée sur cet axe.

A titre de variantes non représentées, la section transversale S₃₀₄, S₃₀₆ du tore précité peut être prévue elliptique. De même, également à titre de variantes non représentées, plutôt que d'être constante suivant l'axe X₃₀₄, X₃₀₆, la section circulaire ou la section elliptique de ce tore peut augmenter ou diminuer suivant l'axe.

En outre, également à titre de variantes, la géométrie incurvée des sections S₁₀₄, S₁₀₆, S₂₀₄, S₂₀₆, S₃₀₄ et S₃₀₆ peut soit être rigoureusement circulaire ou elliptique, soit, au contraire, présenter des variations de courbure suivant sa périphérie. En particulier, comme évoqué plus haut pour la géométrie elliptique des sections S₄ et S₆, la géométrie circulaire ou elliptique des sections S₁₀₄, S₁₀₆, S₂₀₄, S₂₀₆, S₃₀₄ et/ou S₃₀₆ peut présenter une courbure plus petite dans la région centrale des surfaces articulaires correspondantes 104, 106, 204, 206, 304, 306 et/ou présenter une courbure plus grande dans la région périphérique de cette surface articulaire, par rapport au reste de cette section transversale.

Les figures 7 à 10 illustrent ainsi la multiplicité des géométries de construction des surfaces articulaires métacarpienne et scaphoïdienne de l'implant 1 : ces diverses géométries de construction fournissent toutes une mobilité articulaire au corps 2 de l'implant, qui est proche de la mobilité naturelle du trapèze, du moment que les deux surfaces articulaires opposées 4 et 6, 104 et 106, 204 et 206, et 304 et 306 de l'implant 1 sont agencées orthogonalement l'une à l'autre, c'est-à-dire de telle sorte que leurs axes centraux X₄ et X₆, X₁₀₄ et X₁₀₆, X₂₀₄ et X₂₀₆, et X₃₀₄ et X₃₀₆ s'étendent de manière sensiblement perpendiculaire l'un à l'autre. On notera que, dans le cas des axes courbes X₃₀₄ et X_{306,} cette caractéristique de perpendicularité s'apprécie par projection de ces axes dans le plan médian π du corps 2 de l'implant 1, étant remarqué que, pour toutes les formes de réalisation envisagées ci-dessus, au moins les régions centrales respectives des surfaces articulaires métacarpienne et scaphoïdienne sont disposées de part et d'autre de ce plan π. Bien entendu, les projections sur le plan π des axes rectilignes X₄ et X₆, X₁₀₄ et X₁₀₆, et X₂₀₄ et X₂₀₆ sont, elles aussi, perpendiculaires l'une à l'autre.

On comprend également que la géométrie de construction relative à la surface articulaire métacarpienne, choisie parmi toutes celles évoquées ci-dessus, peut être la même, avec un dimensionnement rigoureusement identique ou différent, ou bien être différente de la géométrie de construction choisie pour la surface articulaire scaphoïdienne.

## Revendications

1. Implant d'interposition trapézien (1),
comportant un corps (2) définissant une surface articulaire métacarpienne (4 ; 104 ; 204 ; 304) et une surface articulaire scaphoïdienne (6 ; 106 ; 206 ; 306), qui sont situées de part et d'autre d'un plan médian (π) du corps, qui sont reliées l'une à l'autre par une surface périphérique (8) du corps, dans laquelle est ménagée une gorge périphérique (81) de réception glissante d'un ligament (L) ceinturant partiellement le corps, et qui correspondent chacune à un segment axial de cylindre, de cône ou de tore, ces deux segments axiaux de cylindre, de cône et/ou de tore étant centrés sur des axes respectifs (X₄, X₆; X₁₀₄, X₁₀₆ ; X₂₀₄, X₂₀₆ ; X₃₀₄, X₃₀₆) qui, projetés sur le plan médian (π), sont sensiblement perpendiculaires l'un à l'autre.

2. Implant suivant la revendication 1, **caractérisé en ce que** les surfaces articulaires métacarpienne (4 ; 104 ; 204 ; 304) et scaphoïdienne (6 ; 106 ; 206 ; 306) sont séparées l'une de l'autre, suivant une direction (Z-Z) perpendiculaire au plan médian (π), par une épaisseur minimale (e₂₁) du corps (2) supérieure ou égale à 5 mm.

3. Implant suivant l'une des revendications 1 ou 2, **caractérisé en ce que** la profondeur de la gorge périphérique (81) varie de manière continue entre une valeur maximale, qui est atteinte dans les deux portions de la surface périphérique (8), qui sont opposées l'une à l'autre suivant la direction de l'axe (X₆ ; X₁₀₆ ; X₂₀₆ ; X₃₀₆) associé à la surface articulaire scaphoïdienne (6 ; 106 ; 206 ; 306), et une valeur minimale, qui est atteinte dans les deux portions de la surface périphérique, qui sont opposées l'une à l'autre suivant la direction de l'axe (X₄ ; X₁₀₄ ; X₂₀₄ ; X₃₀₄) associé à la surface articulaire métacarpienne (4 ; 104 ; 204 ; 304).

4. Implant suivant l'une des revendications précédentes, **caractérisé en ce que** l'une ou chacune des surfaces articulaires métacarpienne (4 ; 104 ; 204 ; 304) et scaphoïdienne (6 ; 106 ; 206 ; 306) correspond à un segment axial de cylindre ou de cône ou de tore, à section transversale (S₄, S₆ ; S₁₀₄, S₁₀₆; S₂₀₄, S₂₀₆ ; S₃₀₄, S₃₀₆) incurvée sur toute sa périphérie.

5. Implant suivant la revendication 4, **caractérisé en ce que** l'une ou chacune des surfaces articulaires métacarpienne (4 ; 204 ; 304) et scaphoïdienne (6 ; 206 ; 306) correspond à un segment axial de cylindre ou de cône ou de tore, à section transversale elliptique ou circulaire.

6. Implant suivant l'une des revendications 4 ou 5, **caractérisé en ce que** la section transversale (S₄, S₆) associée à la surface articulaire métacarpienne (4) et/ou à la surface articulaire scaphoïdienne (6) présente, dans la région centrale (42, 62) de cette surface articulaire, une courbure plus petite que le reste de la section transversale.

7. Implant suivant l'une quelconque des revendications 4 à 6, **caractérisé en ce que** la section transversale (S₄, S₆) associée à la surface articulaire métacarpienne (4) et/ou à la surface articulaire scaphoïdienne (6) présente, dans la région périphérique (43, 63) de cette surface articulaire, une courbure plus grande que le reste de la section transversale.

8. Implant suivant l'une quelconque des revendications précédentes, **caractérisé en ce que** la surface articulaire scaphoïdienne (6 ; 106 ; 206 ; 306) présente une dimension (L₆), suivant son axe associé (X₆ ; X₁₀₆ ; X₂₀₆ ; X₃₀₆), supérieure à la dimension (L₄), suivant son axe associé (X₄ ; X₁₀₄ ; X₂₀₄ ; X₃₀₄), de la surface articulaire métacarpienne (4 ; 104 ; 204 ; 304).

9. Implant suivant l'une quelconque des revendications précédentes, **caractérisé en ce que** le corps (2) est dépourvu de moyen de fixation osseuse.

10. Implant suivant l'une quelconque des revendications précédentes, **caractérisé en ce que** l'implant (1) est constitué exclusivement du corps (2) réalisé d'un seul tenant.

## Patentansprüche

1. Trapezbein-Einsatzimplantat (1);
das einen Körper (2) aufweist, der eine Metacarpalgelenkfläche (4; 104; 204; 304) und eine Kahnbein-Gelenkfläche (6; 106; 206; 306) begrenzt, die beidseitig einer medialen Ebene (π) des Körpers angeordnet sind, die miteinander durch eine Umfangsfläche (8) des Körpers verbunden sind, in der eine Umfangsnut (81) zur gleitenden Aufnahme eines Bandes angebracht ist, das teilweise den Körper umschließt, und die jeweils einem axialen Zylinder-, Kegel- oder Torussegment entsprechen, wobei diese zwei axialen Zylinder-, Kegel- und/oder Torussegmente auf jeweilige Achsen (X₄, X₆; X₁₀₄, X₁₀₆; X₂₀₄, X₂₀₆; X₃₀₄, X₃₀₆) zentriert sind, die im Wesentlichen senkrecht zueinander liegen, wenn sie auf die mediale Ebene (π) projiziert werden.

2. Implantat nach Anspruch 1, **dadurch gekennzeichnet, dass** die Metacarpalgelenkfläche (4; 104; 204; 304) und die Kahnbeingelenkfläche (6; 106; 206; 306) gemäß einer Richtung (Z-Z) senkrecht zur Mittelebene (π) durch eine minimale Dicke (e₂₁) des Körpers (2) größer oder gleich 5 mm voneinander getrennt sind.

3. Implantat nach einem der Ansprüche 1 oder 2, **dadurch gekennzeichnet, dass** die Tiefe der Umfangsnut (81) in kontinuierlicher Weise zwischen einem Maximalwert, der in den zwei Bereichen der Umfangsfläche (8) erreicht wird, die gemäß der Richtung der der Kahnbein-Gelenkfläche (6; 106; 206; 306) zugeordneten Achse (X₆; X₁₀₆; X₂₀₆; X₃₀₆) einander gegenüberliegen, und einem Minimalwert, der in den zwei Bereichen der Umfangsfläche erreicht wird, die gemäß der Richtung der der Metacarpal-Gelenkfläche (4; 104; 204; 304) zugeordneten Achse (X₄; X₁₀₄; X₂₀₄; X₃₀₄) einander gegenüberliegen, variiert.

4. Implantat nach einem der vorhergehenden Ansprüche, **dadurch gekennzeichnet, dass** eine oder jede der Metacarpal-Gelenkfläche (4; 104; 204; 304) und Kahnbein-Gelenkfläche (6; 106; 206; 306) einem axialen Zylinder- oder Kegel- oder Torussegment mit über seinem gesamten Umfang gekrümmten Querschnitt (S₄, S₆; S₁₀₄, S₁₀₆; S₂₀₄; S₂₀₆; S₃₀₄, S₃₀₆) entspricht.

5. Implantat nach Anspruch 4, **dadurch gekennzeichnet, dass** eine oder jede der Metacarpal-Gelenkfläche (4; 204; 304) und Kahnbein-Gelenkfläche (6; 206; 306) einem axialen Zylinder- oder Kegel- oder Torussegment mit elliptischem oder kreisförmigem Querschnitt entspricht.

6. Implantat nach einem der Ansprüche 4 oder 5, **dadurch gekennzeichnet, dass** der Querschnitt (S₄, S₆), der der Metacarpal-Gelenkfläche (4) und/oder der Kahnbein-Gelenkfläche (6) zugeordnet ist, in dem Mittelbereich (42, 62) dieser Gelenkfläche eine Krümmung aufweist, die kleiner ist als der Rest des Querschnitts.

7. Implantat nach einem beliebigen der Ansprüche 4 bis 6, **dadurch gekennzeichnet, dass** der Querschnitt (S₄, S₆), der der Metacarpal-Gelenkfläche (4) und/oder der Kahnbein-Gelenkfläche (6) zugeordnet ist, in dem Umfangsbereich (43, 63) dieser Gelenkfläche eine Krümmung aufweist, die größer ist als der Rest des Querschnitts.

8. Implantat nach einem beliebigen der vorhergehenden Ansprüche, **dadurch gekennzeichnet, dass** die Kahnbein-Gelenkfläche (6; 106; 206; 306) eine Abmessung (L₆) gemäß ihrer zugeordneten Achse (X₆; X₁₀₆; X₂₀₆; X₃₀₆) aufweist, die größer ist als die Abmessung (L₄) der Metacarpal-Gelenkfläche (4; 104; 204; 304) gemäß ihrer zugeordneten Achse (X₄; X₁₀₄; X₂₀₄; X₃₀₄).

9. Implantat nach einem beliebigen der vorhergehenden Ansprüche, **dadurch gekennzeichnet, dass** der Körper (2) keine Mittel zur Knochenbefestigung aufweist.

10. Implantat nach einem beliebigen der vorhergehenden Ansprüche, **dadurch gekennzeichnet, dass** das Implantat (1) ausschließlich aus dem Körper (2) besteht, der aus einem einzigen Stück hergestellt ist.

## Claims

1. Trapezium interposition implant (1),
including a body (2) defining a metacarpal articular surface (4; 104; 204; 304) and a scaphoid articular surface (6; 106; 206; 306), which are situated on either side of a median plane (π) of the body, which are connected to each other by a peripheral surface (8) of the body, in which a peripheral groove (81) is formed for slidingly receiving a ligament (L) partially hugging the body, and which each correspond to an axial cylindrical or conical or toroidal segment, said two axial cylindrical or conical and/or toroidal segments being centred on respective axes (X₄, X₆; X₁₀₄, X₁₀₆; X₂₀₄, X₂₀₆; X₃₀₄, X₃₀₆) which, projected onto the median plane (π), are substantially perpendicular to one another.

2. Implant according to claim 1, **characterised in that** the metacarpal (4; 104; 204; 304) and scaphoid (6; 106; 206; 306) articular surfaces are separated from one another, in a direction (Z-Z) perpendicular to the median plane (π), by a minimum thickness (e₂₁) of the body (2) greater than or equal to 5 mm.

3. Implant according to one of claims 1 or 2, **characterised in that** the depth of the peripheral groove (81) varies continuously between a maximum value, which is reached in the two portions of the peripheral surface (8) that are opposite each other in the direction of the axis (X₆; X₁₀₆; X₂₀₆; X₃₀₆) associated with the scaphoid articular surface (6; 106; 206; 306), and a minimum value that is reached in the two portions of the peripheral surface that are opposite each other in the direction of the axis (X₄; X₁₀₄; X₂₀₄; X₃₀₄) associated with the metacarpal articular surface (4; 104; 204; 304).

4. Implant according to one of the preceding claims, **characterised in that** one or each of the metacarpal (4; 104; 204; 304) and scaphoid (6; 106; 206; 306) articular surfaces corresponds to an axial cylindrical or conical or toroidal segment, with a cross section (S₄, S₆; S₁₀₄, S₁₀₆; S₂₀₄, S₂₀₆; S₃₀₄, S₃₀₆) that is curved over its entire periphery.

5. Implant according to claim 4, **characterised in that** one or each of the metacarpal (4; 204; 304) and scaphoid (6; 206; 306) articular surfaces corresponds to an axial cylindrical or conical or toroidal segment, with an elliptical or circular cross section.

6. Implant according to one of claims 4 or 5, **characterised in that** the cross section (S₄, S₆) associated with the metacarpal articular surface (4) and/or the scaphoid articular surface (6) has, in the central region (42, 62) of said articular surface, a smaller curvature than the rest of the cross section.

7. Implant according to any one of claims 4 to 6, **characterised in that** the cross section (S₄, S₆) associated with the metacarpal articular surface (4) and/or the scaphoid articular surface (6) has, in the peripheral region (43, 63) of said articular surface, a larger curvature than the rest of the cross section.

8. Implant according to any one of the preceding claims, **characterised in that** the scaphoid articular surface (6; 106; 206; 306) has a dimension (L₆), along its associated axis (X₆; X₁₀₆; X₂₀₆; X₃₀₆), larger than the dimension (L₄), along its associated axis (X₄; X₁₀₄; X₂₀₄; X₃₀₄), of the metacarpal articular surface (4; 104; 204; 304).

9. Implant according to any one of the preceding claims, **characterised in that** the body (2) has no bone fastening means.

10. Implant according to any one of the preceding claims, **characterised in that** the implant (1) is made up exclusively of the body (2) made in a single piece.
